(19) Europäisches Patentamt
European Patent Office
Office européen des brevets

(11) **EP 2 771 670 B1**

(12) **FASCICULE DE BREVET EUROPEEN**

(45) Date de publication et mention de la délivrance du brevet:
**26.07.2023 Bulletin 2023/30**

(21) Numéro de dépôt: **12778807.3**

(22) Date de dépôt: **17.10.2012**

(51) Classification Internationale des Brevets (IPC):
**G01N 21/35** (2014.01)          **G01N 33/28** (2006.01)

(52) Classification Coopérative des Brevets (CPC):
**G01N 33/2888; G01N 21/3577; G01N 21/552;**
G01N 21/3554; G01N 2201/1293

(86) Numéro de dépôt international:
**PCT/IB2012/055667**

(87) Numéro de publication internationale:
**WO 2013/061216 (02.05.2013 Gazette 2013/18)**

(54) **PROCEDE ET APPAREIL DE CARACTERISATION D'UN FLUIDE HYDRAULIQUE.**

VERFAHREN UND VORRICHTUNG ZUR CHARAKTERISIERUNG EINER HYDRAULISCHEN FLÜSSIGKEIT

METHOD AND APPARATUS FOR THE CHARACTERISATION OF A HYDRAULIC FLUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.10.2011 FR 1159630**

(43) Date de publication de la demande:
**03.09.2014 Bulletin 2014/36**

(73) Titulaire: **Appareillages et Bancs Hydrauliques Chatelleraudais**
**86100 Chatellerault (FR)**

(72) Inventeurs:
• **PIERRE-LOTI-VIAUD, Daniel**
**F-91740 Chalou Moulineux (FR)**
• **RICHARD, Jean-Paul**
**F-75007 Paris (FR)**
• **RICHARD, Christian**
**F-86100 Chatellerault (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A2-2009/082418**

• **"Lubricant Condition Monitoring Using FluidScan IR Spectroscopy - Determining Lubricant Degradation by Total Acid Number (TAN) or Oxidation by Spectro", , 3 mai 2006 (2006-05-03), XP055020636, Extrait de l'Internet: URL:http://www.azom.com/article.aspx?ArticleID=4580 [extrait le 2012-02-29]**
• **ANA M. AGUILERA ET AL: "Using basis expansions for estimating functional PLS regression", CHEMOMETRICS AND INTELLIGENT LABORATORY SYSTEMS, vol. 104, no. 2, 1 décembre 2010 (2010-12-01), pages 289-305, XP055020738, ISSN: 0169-7439, DOI: 10.1016/j.chemolab.2010.09.007**
• **Alessandra Borin ET AL: "Multivariate quality control of lubricating oils using Fourier transform infrared spectroscopy", JOURNAL OF THE BRAZILIAN CHEMICAL SOCIETY, vol. 15, no. 4, 1 January 2004 (2004-01-01), pages 570-576, XP055405934, São Paulo; BR ISSN: 0103-5053, DOI: 10.1590/S0103-50532004000400020**

**Description**

L'invention porte sur un procédé de caractérisation d'un fluide hydraulique, et en particulier un procédé pour la détermination de l'état de dégradation du fluide hydraulique (« huile ») de type ester phosphate contenu dans les circuits hydrauliques d'un avion. L'invention porte également sur un appareil pour la mise en oeuvre d'une telle méthode.

Les avions, et notamment les avions de ligne, comprennent plusieurs circuits d'actionnement hydraulique (pour le levage du train d'atterrissage, la commande des volets hypersustentateurs, etc.), contenant des huiles qui sont souvent de type ester phosphate. Ces huiles sont sujettes à diverses dégradations, notamment liées à l'absorption d'eau ou la présence de composés chlorés, qui affectent leurs propriétés physiques et physico-chimiques telles que la viscosité, la densité (masse volumique) et l'acidité. En effet, les esters phosphates réagissent avec l'eau selon une réaction du type :

$$\text{Esters phosphates + eau} \leftrightarrow \text{Acide phosphorique + alcools.}$$

Une huile qui ne respecte certaines normes concernant les propriétés physico-chimiques évoquées précédemment doit être changée rapidement, car un avion dont l'huile n'est pas conforme peut être soumis à une interdiction de voler. Un exemple de norme couramment utilisée pour la qualité d'une huile aéronautique ester phosphate est la suivante :

- teneur en eau $\leq$ 5000 ppm (parts par million)
- teneur en chlore $\leq$ 200 ppm
- indice d'acidité $\leq$ 1,5 mgKOH/g
- conductivité $\leq$ 0,3 $\mu$S/cm
- viscosité à 38°C : 6 à 12,5 Cst
- densité à 25°C : 0,970 à 1,066 g/cm$^3$

Cependant, le prix élevé des huiles hydrauliques incite à ne les changer que lorsque cela est réellement nécessaire.

La procédure généralement utilisée consiste à prélever des échantillons d'huile (de l'ordre de 250 ml) et les envoyer à des laboratoires spécialisés qui effectuent des analyses physico-chimiques. Les résultats sont obtenus en quelques jours, et l'avion est immobilisé pendant ce temps, ce qui a un coût important qui s'ajoute au coût des analyses elles-mêmes.

Il existe donc un besoin pour un procédé de caractérisation d'un fluide hydraulique, en particulier de type ester phosphate, permettant de discriminer entre un échantillon d'une fluide conforme à l'usage et un échantillon d'un fluide à changer ou à régénérer, qui soit plus rapide et moins couteux que les procédé de laboratoire conventionnels.

L'article de S. Paul et al. « Chemical Contamination Sensor for Phosphate Ester Hydraulic Fluids », International Journal of Aerospace Engineering, Vol. 2010, Article ID 156281, doi: 10.1155/2010/156281 décrit un procédé spectroscopique de caractérisation d'un fluide hydraulique de type ester phosphate, basé sur une mesure d'absorbance à quatre longueurs d'onde dans l'infrarouge. Ce procédé ne permet de déterminer que la teneur en eau et l'indice d'acidité de l'huile, ce qui n'est pas suffisant. Par ailleurs, le procédé ne permet pas de déterminer de manière quantitative des indices d'acidité supérieurs à 1 mgKOH/g, ce qui peut conduire à écarter des huiles encore conformes aux standards d'utilisation (indice d'acidité compris entre 1 et 1,5 mgKOH/g).

L'article de N. Robinson « Monitoring oil dégradation with infrared spectroscopy », Wearcheck Technical Bulletin n°18 décrit l'application de techniques spectroscopiques au contrôle de dégradation des huiles lubrifiantes, et en particulier à la détermination de l'indice de basicité (« Total Base Number », TBN) par application d'une régression par moindres carrés partiels (PLS) ou en composantes principales (PCR) à un spectre d'absorption infrarouge.

WO 2009/082418 A2 (D1) décrit une méthode pour prédire la viscosité d'une huile de moteur en corrélant des valeurs de viscosité de l'huile mesurées par des méthodes conventionnelles (ASTM) à des pics de spectres Raman obtenus avec l'huile à caractériser.

L'article (D2) intitulé "Lubricant Condition Monitoring Using FluidScan IR Spectroscopy Determining Lubricant Dégradation by Total Acid Number (TAN) or Oxidation by Spectro" publié en ligne sur le site www.azom.com le 3 mai 2006, décrit un appareil portatif (FluidScan) permettant de déterminer le niveau de dégradation d'une huile en mesurant son acidité ou son oxydation. Cet appareil met en oeuvre une technique d'analyse de données spectrales infrarouge par régression en composantes principales pour calculer le degré d'acidité de l'huile.

Dans l'article scientifique (D3) intitulé « Using basis expansions for estimating functional PLS régression », publié dans Chemometrics and Intelligent Laboratory Systems, Vol. 104, no. 2, pages 289-305, en date du 01/12/2010, Ana M. AGUILERA *et al.* comparent la méthode de régression par moindres carrés partiels (PLS : *Partial Least Squares Regression*) aux méthodes d'analyse en composantes principales (PCR : *Principal Component Regression*) et d'analyse en composantes principales fonctionnelles (FPCA : *Functional Principal Component Analysis*) en s'appuyant sur de l'analyse spectrale.

Dans l'article scientifique (D4) intitulé « Multivariate quality control of lubricating oils using Fourier transform infrared spectroscopy », publié dans Journal of the Brazilian Chemical Society, vol. 15, n° 4, pages 570-576, en date du 01/01/2004, Alessandra BORIN *et al.* décrivent une méthode d'analyse en composantes principales (ACP) pour qualifier une huile de moteur de manière à déterminer la catégorie d'huile.

L'invention vise à remédier aux inconvénients précités de l'art antérieur.

Une idée à la base de la présente invention est que la plupart de l'information contenue dans les spectres ne se trouve pas dans les pics « majeurs », facilement identifiables, mais bien dans la partie continue du spectre et dans les « petits » pics, qui sont le plus souvent négligés. C'est donc l'intégralité du spectre dans une ou plusieurs régions spectrales qui doit donc être utilisé pour la caractérisation du fluide. Pour ce faire, il est proposé d'avoir recours à une technique de réduction dimensionnelle, qui permet de représenter - avec une perte d'information connue et généralement petite - un spectre par un vecteur constitué par un nombre limité (généralement moins vingt et de préférence dix ou moins) de paramètres représentatifs. La technique de réduction dimensionnelle - ou « vectorisation » - qui s'est avérée être la mieux adaptée est l'analyse en composantes principales fonctionnelle, ou ACPF, en particulier dans sa forme régularisée. D'autres techniques d'analyse statistique fonctionnelle peuvent être utilisées dans des procédés qui ne font pas partie de l'invention revendiquée, et notamment des techniques de type non paramétrique. On peut citer à ce propos une approche basée sur la décomposition en ondelettes des spectres ; cette technique s'apparente à l'ACPF en ce que chaque spectre est réduit à un nombre très limité de paramètres estimés par projection sur une base de $L^2([a,b])$, dite base d'ondelette. Cette base comportant évidemment un nombre infini d'élément, il faut déterminer quelles composantes sont les plus importantes relativement aux données. L'estimation statistique porte alors à la fois sur le nombre de composantes à garder (seuillage) et sur le calcul de ces composantes. Cette technique, utilisée notamment en traitement du signal, permet de compresser les données de façon très significative dans un grand nombre de cas. Elle diffère de l'ACPF en ce qu'elle ne tient pas compte des données pour déterminer la base de $L^2$, qui est fixée a priori par le biais d'ondelettes père et mère. Aucune hypothèse sur la structure des spectres n'intervient donc, ce qui confère un avantage à l'ACPF dans le cas particulier de l'invention problème. Voir à ce propos l'ouvrage de Larry Wasserman «All of Nonparametric Statistics», Springer 2006, et en particulier son paragraphe 9.3.

Les techniques fonctionnelles, notamment régularisées, s'avèrent plus performantes que les techniques multivariées telles que l'ACP ou la PLS, et cela en particulier lorsque les spectres présentent un grand nombre de petits pics difficilement distinguable du bruit de fond.

Ensuite, des techniques de régression multiple et/ou de classification peuvent être appliquées à ce vecteur pour quantifier les paramètres physico-chimiques de l'huile, ou pour déterminer directement sa conformité ou non-conformité à une norme prédéfinie.

Ainsi, un objet de l'invention est un procédé de caractérisation d'un fluide hydraulique par application à un spectre d'un échantillon du fluide à caractériser d'une technique d'analyse statistique fonctionnelle pour déterminer une pluralité de grandeurs physico-chimiques dudit fluides.

L'invention est définie par les revendications indépendantes.

- L'application de ladite technique d'analyse statistique peut comprendre :

  - le découpage dudit spectre en une pluralité de portions ;

    - le calcul, par analyse en composantes principales fonctionnelle, d'un vecteur de paramètres représentatifs de chaque dite portion ; et
    - la concaténation des vecteurs de paramètres ainsi obtenus.

- Après l'application de ladite technique d'analyse statistique, il peut être prévu de déterminer si ledit spectre doit être écarté par application de la méthode de Wilks audit vecteur de paramètres.
- Ladite technique d'analyse statistique peut être mise en oeuvre en décomposant ledit spectre sur une base de fonctions propres déterminée à partir d'une base de données de spectres de référence.
- Les valeurs desdites grandeurs physico-chimiques peuvent être calculées par régression multivariée l'aide de coefficients de régression déterminée à partir d'une base de données de spectres de référence.
- En variante ou en complément, le procédé peut comprendre également une opération de classification pour attribuer ledit spectre à une classe d'échantillons, lesdits coefficients de régression dépendant de ladite classe.
- Le spectre dudit échantillon peut être ajouté à ladite ou au moins une dite base de données de spectres des référence.
- Le procédé peut comporter le fractionnement de l'échantillon à caractériser en une pluralité de sous-échantillons, l'acquisition de deux spectres d'absorption de chaque sous-échantillons de manière à obtenir une pluralité de paires de spectres, et la détermination de régions de confiance desdites grandeurs physico-chimiques par application d'un test circulaire sur les vecteurs de paramètres caractéristiques obtenus pour lesdites paires de spectres. Ledit test circulaire peut comprendre la détermination d'un ellipsoïde de confiance de répétabilité et d'un ellipsoïde de confiance de reproductibilité, et la sélection des seules spectres caractérisés par une répétabilité et une reproductibilité considérées comme suffisantes. Ladite sélection des seuls spectres caractérisés par une répétabilité et une reproductibilité considérées comme suffisantes peut être itérée.

- Le procédé peut comporter également, préalablement à l'application de ladite technique d'analyse statistique, une étape de prétraitement dudit spectre comprenant la soustraction d'une moyenne lissée, calculée à partir d'une base de données de spectres de référence.
- Préalablement à l'application de ladite technique d'analyse statistique, il peut être prévu de déterminer si ledit spectre doit être écarté par mesure de l'aire sous-tendue par un pic ou massif prédéterminé du spectre.
- Lesdites grandeurs physico-chimiques peuvent comprendre au moins un parmi : la teneur en eau, un indice d'acidité, la conductivité, la viscosité, la densité, la teneur en chlore. Plus particulièrement elles peuvent comprendre la teneur en eau, un indice d'acidité, la conductivité, la viscosité et la densité.
- Le spectre acquis peut être transmis, par l'intermédiaire d'un système de télécommunications, à un serveur qui met en oeuvre ladite technique d'analyse statistique.
- Ledit spectre peut être acquis au moyen d'un équipement embarqué sur un avion, relié à un circuit hydraulique de ce dernier.

L'invention porte également sur l'application d'un tel procédé à la détermination de l'état de dégradation du fluide hydraulique de type ester phosphate d'un avion, ainsi que sur un appareil pour la mise en oeuvre d'un tel procédé.

D'autres caractéristiques, détails et avantages de l'invention ressortiront à la lecture de la description faite en référence aux dessins annexés donnés à titre d'exemple et qui représentent, respectivement :

- La figure 1, le schéma d'un appareil pour la mise en oeuvre d'un procédé selon un mode de réalisation de l'invention ;
- La figure 2, des spectres infrarouges d'absorption d'une pluralité d'échantillons d'une huile hydraulique de type ester phosphate ;
- La figure 3, les mêmes spectres reconstitués après vectorisation ACPF ; et
- Les figures 4 à 7 des courbes de régression pour la teneur en eau, l'indice d'acidité, la conductivité et la teneur en chlore desdits échantillons.

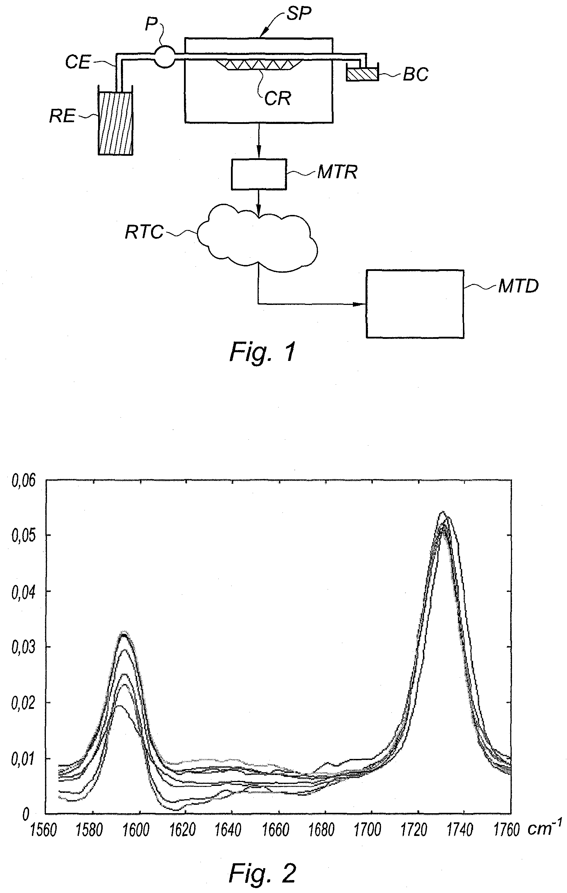

Le dispositif de la figure 1 comprend un récipient RE destiné à contenir un échantillon du fluide hydraulique à caractériser, typiquement d'une contenance de quelques centaines de millilitres. Le fluide est aspiré dans un conduit CE au moyen d'une pompe volumétrique P, et amené dans un spectromètre infrarouge SP. Il peut s'agir par exemple d'un spectromètre infrarouge à transformé de Fourier du type à réflexion totale atténuée (FTIR-ATR), par exemple le spectromètre Alpha de la société Bruker. Un tel spectromètre comprend un cristal CR transparent à l'infrarouge, dont une surface est mise en contact avec le fluide. Un faisceau de lumière infrarouge se propage à l'intérieur du cristal et subit une réflexion interne totale sur la surface mouillée par le fluide. Une onde évanescente s'étend à l'intérieur du fluide, sur une faible distance, et est partiellement absorbée, ce qui se traduit par une atténuation de la réflexion totale. L'analyse spectrale du rayonnement réfléchi se fait au moyen d'un interféromètre, sur la base du principe de la spectroscopie par transformée de Fourier. D'autres types de spectromètres peuvent aussi être utilisés.

Le fluide quitte ensuite le spectromètre et est amené dans un bac de collecte BC ; il peut ensuite être analysé selon une technique conventionnelle afin d'étalonner le dispositif de l'invention, ou dans un but de vérification ; ou bien être éliminé.

Le spectre d'absorption acquis par le spectromètre est numérisé et converti en un fichier informatique d'un format prédéfini. Un moyen de transmission MTR transmet ce fichier, par l'intermédiaire d'un réseau de télécommunications (téléphone, internet...), à un moyen de traitement des données MTD - généralement un ordinateur programmé de manière opportune - qui en extrait les informations physico-chimiques recherchées. En pratique l'utilisateur (compagnie aérienne, aéroport...) détient seulement la partie de l'appareil de l'invention qui permet d'acquérir, mettre en forme et transmettre les spectres ; le traitement des données est effectué de manière centralisée par un fournisseur de services. Cela permet, notamment, de perfectionner sans cesse le traitement, par exemple en enrichissant la base de données sur laquelle se basent la vectorisation et la régression.

Bien entendu, il est également possible d'effectuer une partie du traitement (notamment le prétraitement, ou conditionnement, des spectres), voire la totalité, en local.

En variante, le dispositif selon un mode de réalisation peut être embarqué sur un avion ; dans ce cas le récipient RE et le bac de collecte BC peuvent être remplacés par des piquages sur un circuit hydraulique. Dans ce cas également, le traitement des données peut se faire en local ou à distance, de manière centralisée.

La figure 2 montre un ensemble de spectres d'absorption de plusieurs échantillons d'une huile hydraulique aéronautique de type ester phosphate ; l'axe des abscisses représente le nombre d'onde en cm$^{-1}$, l'axe des ordonnées l'absorbance en unités arbitraires ; dans un souci de simplicité, seule la partie des spectres correspondant à des nombres d'onde compris entre 1560 et 1760 cm$^{-1}$ est représentée. Les spectres présentent deux pics principaux centrés à 1590

et 1740 cm$^{-1}$ et, entre les deux, une région ne présentant pas de pics bien définis. C'est principalement cette région qui est utilisée par le procédé de caractérisation proprement dit. L'aire sous-tendue par des pics principaux (notamment un massif de pics situé autour de 3500 cm$^{-1}$) peut être utilisée pour détecter des spectres aberrants, à écarter : en effet, une aire trop faible indique vraisemblablement un défaut dans la mesure ou son environnement.

Chaque spectre d'absorption qui n'est pas écarté en tant qu'aberrant est soumis à un prétraitement ou conditionnement qui comporte, notamment, la soustraction d'un spectre moyen lissé obtenu à partir d'une base de données de spectres de référence. Cette moyenne lissée peut être obtenue en calculant une interpolation spline, comme expliqué aux chapitres 2 et 3 de l'ouvrage de P. J. Green, et B. W. Silverman « Nonparametric Regression and Generalized Linear Models - a roughness penalty approach », 1e éd., Chapman & Hall, Londres, 1994. La soustraction de la moyenne met en évidence les différences entre les spectres ; ce sont en effet ces différences qui contiennent l'information recherchée. L'utilisation d'une moyenne lissée permet d'éviter les discontinuités dues aux erreurs aléatoires et qui apparaissent nécessairement dans une moyenne ordinaire.

Soit l'intervalle [a; b] et $\{t_j, j \in [1, p]\}$ tels que $a < t_1 < ... < t_p < b$.

Chaque point d'un spectre peut être représenté par une suite de points $X^j$. Soit $\overline{X^j}$ la moyenne point par point des différents spectres ; la moyenne lissée est la fonction $\hat{\mu}(t)$ qui minimise :

$$\sum_{j=1}^{p} \left( \overline{X^j} - \mu(t_j) \right)^2 + \alpha \int_{[t_1, t_p]} \mu''(t)^2 dt$$

où $\mu''$ est la dérivée seconde de $\mu(t)$.

Le paramètre de régularisation a peut être déterminé de manière automatique, par une technique de validation croisée décrite en détail dans le chapitre 3 de l'ouvrage précité de P. J. Green, et B. W. Silverman. L'idée générale est la suivante : pour $\alpha$ fixé on calcule la moyenne lissée $\hat{\mu}^{-i}(t)$ en ignorant le point $\overline{X^j}$ et on utilise cette moyenne pour prédire le point ignoré ; puis on répète cette opération pour tous les points $\overline{X^j}$ et calcule la moyenne quadratique des erreurs de prédiction correspondants, $CV(\alpha)$. La valeur optimale du paramètre de régularisation est celle qui minimise $CV(a)$.

Les spectres ainsi conditionnés sont ensuite découpés en portions, ou zones (typiquement : 5 à 10 portions, par exemple 8), de manière à obtenir des fichiers de plus petite taille, plus maniables.

On réalise ensuite une analyse en composantes principales fonctionnelle (ACPF), de préférence régularisée, de chaque portion du spectre. L'ACPF est l'extension de l'analyse par composante principales au cas où les données sont représentées par des fonctions continues (ici, des spectres). Bien entendu, pour qu'un traitement par ordinateur soit possible, les fonctions continues doivent être discrétisées, et converties en vecteurs. L'expression « analyse en composantes principales fonctionnelle » est utilisée au sens large, pour indiquer que ces vecteurs sont de grandes dimensions (typiquement, plusieurs centaines ou milliers de points) et distinguer le procédé selon ce mode préféré de réalisation de l'invention du cas où l'on ne considère que les absorbances à un nombre limité de longueurs d'onde, correspondant généralement à des pics bien identifiés.

Le principe de l'ACPF est d'approcher une fonction (un spectre) par une combinaison linéaire de fonctions formant une base orthonormée, choisie de manière à minimiser l'erreur quadratique. On trouve que la base optimale est formée par les fonctions propres de l'opérateur (la matrice, dans le cas discret) de covariance, estimée à partir de ladite base de données de spectres de référence. Il est intéressant que même après la phase initiale d'étalonnage on peut se servir des spectres des échantillons à caractériser pour affiner le calcul de ces fonctions propres. Comme cela sera expliqué plus loin, des techniques statistiques appropriées permettent d'identifier les spectres aberrants ; ainsi, seuls les spectres représentatifs seront retenus pour enrichir progressivement la base des données.

Dans l'ACPF régularisée, on introduit un terme de pénalisation proportionnel à la dérivée seconde des fonctions propres.

D'un point de vue plus mathématique, soit V la matrice de variance-covariance donnée par :

$$V_{ij} = cov(X^i, X^j) = \frac{1}{n} \sum_{k=1}^{n} (X_k^i - \hat{\mu}(t_i)) (X_k^j - \hat{\mu}(t_j))$$

où l'indice k identifie les spectres de la base de données. Pour déterminer la première fonction propre, on recherche donc la fonction $\phi^1 : [t_1; t_p] \to \Re$ de norme euclidienne 1 et vérifiant, pour $\alpha > 0$ :

$$\phi^T V \phi - \alpha \int_{t_1}^{t_p} (\phi''(s))^2 ds = \max_{\|\psi\|=1} \left(\psi^T V \psi - \alpha \int_{t_1}^{t_p} (\psi''(s))^2 ds\right)$$

Comme pour le calcul de la moyenne lissée, $\alpha$ peut être déterminé de manière automatique par validation croisée. On démontre que $\phi^1$ est une *spline.*

Soient :

- pour i=1 ... n-1 : $h_i = t_{i+1} - t_i$;
- Q la matrice de taille p, (p-2) dont les éléments sont:
  $q_{j-1,j} = 1/h_{j-14}$ ; $q_{j,j} = -1/h_{j-1} - 1/h_j$; $q_{j+1,j} = 1/h_j$; $q_{i,j} = 0$ pour $|i-j| > 1$ ;
- R la matrice symétrique de dimensions (p-2), (p-2) dont les éléments sont, pour i et j allant de 2 à p-1 :
- $r_{i,i} = (h_{i-1} + h_i)/3$ ; $r_{i,i+1} = h_i/6$; $r_{i,j} = 0$ pour $|i-j| > 1$.

On définit K par $K = QR^{-1}Q^T$, l'exposant T indiquant la transposée.

On démontre que $\phi^1$ est la plus grande valeur propre de $V - \alpha_1 K$, avec $\alpha_1$ déterminé par validation croisée comme expliqué plus haut.

Puis on détermine la deuxième fonction propre par une optimisation sous contrainte d'orthogonalité avec $\phi^1$:

$$\phi^T (V - \alpha K) \phi = \max_{\|\psi\|=1 \; et \; \psi^T \phi^1 = 0} \left(\psi^T (V - \alpha K) \psi\right)$$

où le paramètre de régularisation $\alpha$ prend une valeur $\alpha_2$ déterminé à son tour par validation croisée, et ainsi de suite.

Pour plus de détails sur l'ACPF on peut se rapporter au chapitre 8 du livre de J.O. Ramsay et B.W. Silverman « Functional Data Analysis », 2e édition, Springer-Verlag, 2006. Plusieurs techniques de régularisation existent ; celle décrite ci-dessus a été développée par Green et Silverman (voir le livre précité de ces auteurs). Une autre approche est décrite par le chapitre 9 du livre précité de J.O. Ramsay et B.W. Silverman.

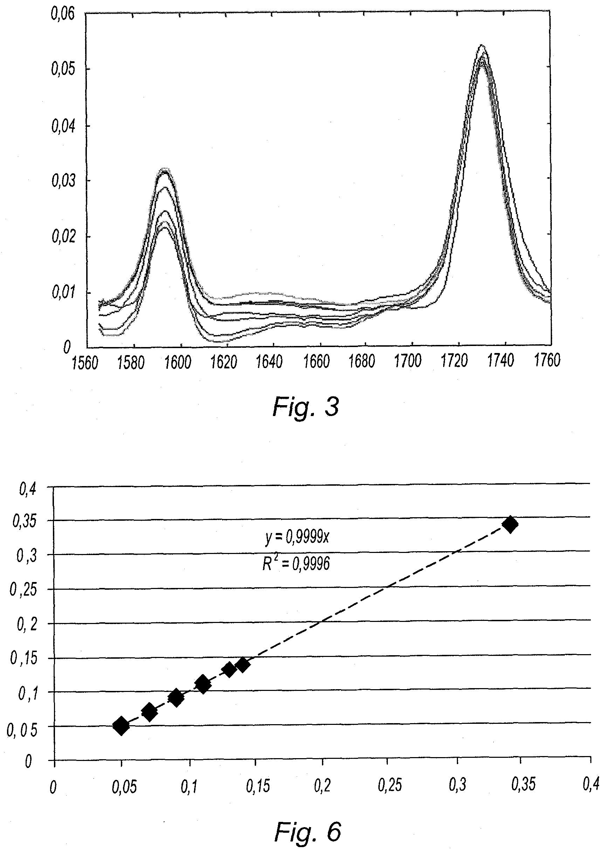

Au final, chaque portion de spectre peut être représentée de manière approchée par un vecteur constitué par les premiers coefficients de la décomposition en fonctions propres de l'ACPF (on parle donc de « vectorisation »). Un petit nombre (généralement inférieur à 10, par exemple égal à 4 ou 5) est généralement suffisant pour obtenir une bonne approximation. Puis, les vecteurs associés à chaque portion de spectre sont concaténés entre eux pour former un vecteur plus grand, mais toujours beaucoup plus petit que le spectre complet. La figure 3 montre les spectres de la figure 2, reconstitués à partir d'une dizaine de composantes principales et de la moyenne lissée.

Il est important de noter que l'on peut combiner par concaténation des vecteurs représentatif issus de portions disjointes du spectre d'absorption, notamment dans d'autres régions spectrales que l'infrarouge (par exemple dans le visible-proche ultraviolet, région sensible aux alcools et aux phénols), ou même associer des vecteurs issus d'autres mesures physiques. Mais, comme cela sera montré ci-après, la prise en compte des spectres d'absorption infrarouges entre 600 et 3600 cm$^{-1}$ environ suffit pour caractériser les huiles hydrauliques ester phosphates de manière satisfaisante.

Une fois effectuée la vectorisation, il est possible d'effectuer une nouvelle étape de détection d'échantillons aberrants par un test statistique (par exemple un test de Wilks) - voir à ce propose H. Kres « Statistical Tables for Multivariate Analysis », table 13, pages 235 et suivantes. Le principe de ce test est de considérer aberrants et éliminer les échantillons qui sont trop éloignés, selon une métrique déterminée, d'un ensemble d'échantillons de référence. Comme pour la détermination des fonctions propres de l'ACPF, la base de données d'échantillons de référence pour le test statistique peut être enrichie progressivement en y intégrant les échantillons considérés comme non aberrants.

Une alerte peut être émise si l'échantillon est considéré aberrant. Cela peut indiquer, par exemple, que l'échantillon n'est pas du type attendu (par exemple, il ne s'agit pas d'une huile hydraulique), ou alors que l'appareil d'analyse ne fonctionne pas correctement (par exemple, le spectromètre est désaligné).

Ensuite, on effectue une régression multiple pour déterminer des valeurs numériques des paramètres physico-chimiques recherchés en fonction du vecteur obtenu par ACPF. Les coefficients de régression sont déterminés à partir des échantillons de référence, dont les propriétés sont supposées connues (mesurées par des analyses de laboratoire conventionnelles). La base de données des échantillons de référence peut être enrichie au cours du temps.

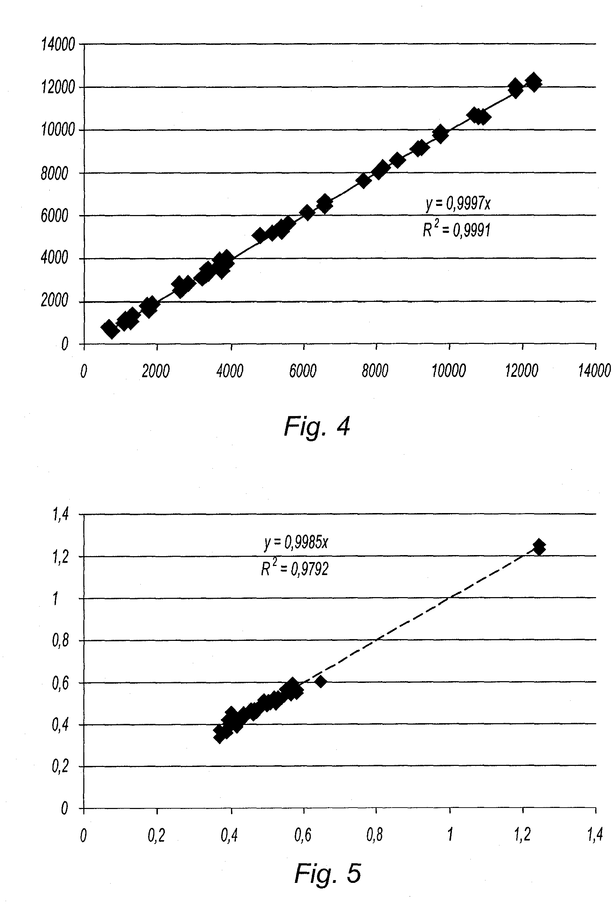

Les figures 4 à 6 montrent les droites de régression linéaire pour la teneur en eau (en ppm, parties par million), l'indice d'acidité (en mgKOH/g), la conductivité (0,3 $\mu$S/cm), respectivement. L'axe des abscisses donne les valeurs obtenues par des mesures de laboratoire conventionnelles, et l'axe des ordonnées les valeurs prédites par la méthode de l'invention. On peut vérifier que tant la pente de la droite de régression que le coefficient de corrélation linéaire $R^2$

sont très proches de 1, ce qui est très satisfaisant.

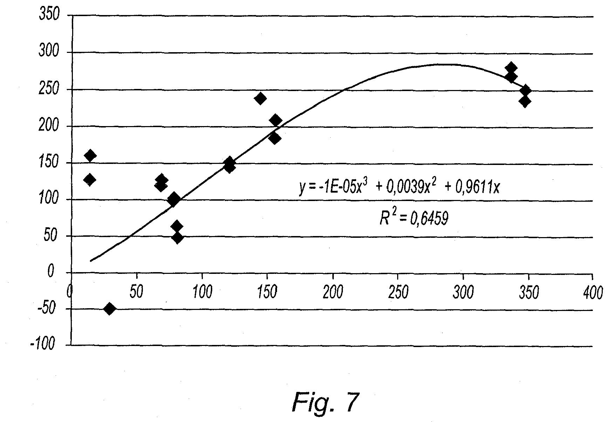

Comme on peut le voir sur la figure 7, pour la teneur en chlore (en ppm) on doit recourir à une régression non linéaire, et il est plus difficile d'obtenir une estimation quantitative de ce paramètre. Il reste possible d'identifier les échantillons qui présentent une teneur en chlore supérieure à la limite autorisée, en acceptant quelques faux positifs.

En variante, il est possible de ne pas effectuer une régression multiple mais une simple classification, pour distinguer les échantillons « conformes » de ceux qui sont « non conformes ». Ainsi, on ne détermine pas les paramètres physico-chimiques d'intérêt individuellement et de manière quantitative, mais conjointement et de manière plus qualitative.

Des nombreuses techniques de classifications sont connues de l'art antérieur.

Il est également possible d'effectuer une classification préalable à la régression. Ainsi, la base de données peut comprendre des échantillons de références regroupés en classes (par exemple, associés à différents producteurs d'huile), des coefficients de régression étant déterminés pour chaque classe. Un algorithme de classification attribue le vecteur représentatif du spectre d'un échantillon à caractériser à une dite classe, puis les coefficients de régression correspondants sont appliqués.

Des mesures, quelle que soit la méthode par laquelle elles ont été obtenues, doivent en général être accompagnées de leur intervalles de confiance. Plus précisément, on distingue :

- la *justesse,* qui indique si la mesure observée est proche de la valeur vraie (ou supposée telle) ;
- la *répétabilité,* qui donne la dispersion des mesures effectuées dans des conditions expérimentales quasiment identiques, ou, au moins, très proches ;
- la *reproductibilité,* qui donne la dispersion des mesures effectuées dans des conditions différentes, suivant le même protocole expérimental.

Dans chaque cas, on définit des intervalles, ou zones, de confiance.

On appelle zone de confiance à N% la dispersion des résultats de mesures concernant N% des échantillons mesurés. A titre d'exemple, on suppose d'effectuer 100 mesures de teneur en eau sur un même échantillon d'huile avec la même méthode : 95% de ces mesures se situent entre 2500 et 2600 ppm ; 3 mesures sont au-delà de 2700 ppm et 2 en-deçà de 2400 ppm. On dira que la zone de confiance, pour cette mesure avec cette méthode, est 2500 à 2600 ppm, à 95%. Si, et seulement si, la répartition est gaussienne (loi normale), alors la zone de confiance à 95% est de la forme : $m \pm 1{,}96\sigma$ où m est la moyenne expérimentale et $\sigma$ l'écart-type expérimental. L'hypothèse gaussienne est généralement implicite.

L'ensemble des conditions de répétabilité et de reproductibilité constituent la fidélité des mesures. La justesse et la fidélité définissent l'exactitude.

La fidélité est normalisée par la norme ISO5725, lorsqu'une seule valeur, scalaire, définit une mesure. C'est l'approche généralement utilisées dans les méthodes d'analyse connues de l'art antérieur : les zones de confiance des différents paramètres physico-chimiques sont déterminées indépendamment les unes des autres. Une approche beaucoup plus intéressante consiste à déterminer des ellipsoïdes de confiance pour la répétabilité et la reproducibilité, ce qui permet de prendre en compte le fait que les différents paramètres ne sont pas réellement indépendants les uns des autres. Cette approche à été suggérée dans l'article de J. P. Richard, P. Deheuvels et D. Pierre-Loti-Viaud « Une approche nouvelle pour l'exploitation des données chromatographiques », Ann. Fals. Exp. Chim. 92, 455 - 470 (2000). Le procédé et l'appareil de l'invention conviennent particulièrement bien à sa mise en oeuvre.

Concrètement, il est possible de réaliser un « test circulaire » (appelé aussi « ring test » ou « coopérative study » dans la littérature en langue anglaise) avec l'appareil de la figure 1 de la manière suivante.

La pompe P est utilisée pour déposer une goutte d'un échantillon de fluide hydraulique sur le cristal CR du spectromètre. Deux spectres de cette goutte sont acquis avec un intervalle de temps fixé entre les deux acquisitions. Ces deux mesures successives sont assimilées à deux mesures de répétabilité effectuées dans un seul laboratoire, selon la technique, connue en soi, des échantillons appariés. Puis on actionne à nouveau la pompe pour évacuer la goutte ayant déjà fait l'objet d'une double acquisition, et on dépose une nouvelle goutte sur le cristal du spectromètre, et ainsi de suite. Un rinçage avec un liquide « neutre » (par exemple, de l'isopropanol) peut être prévu entre deux acquisitions.

Ainsi, on acquiert une pluralité (par exemple, 25) de paires de spectres. Chacune desdites paires est assimilée à un laboratoire au sens de la norme ISO5725 et de sa généralisation mentionnée par l'article précité de J.P. Richard et al.

En comparant les paires d'acquisitions successives il est possible de détecter des dérives, qui peuvent être dues à un dysfonctionnement du spectromètre ou bien à une insuffisante d'homogénéité de l'échantillon (à cause, notamment, d'une décantation suivie par une agitation insuffisante). Si ces dérives dépassent un seuil statistique, une alerte peut être émise.

En outre, les différentes paires de mesure permettent de définir un ellipsoïde de confiance pour la répétabilité, et donc de sélectionner seulement certaines paires de spectres (cela correspond à la sélection, dans un test circulaire classique, des laboratoires compétents). Puis un ellipsoïde de confiance pour la reproductibilité est déterminé à partir des seules paires retenues après l'étape précédente ; on procède ainsi à une nouvelle sélection des spectres acquis. Les spectres retenus après cette double sélection sont utilisés pour calculer, par vectorisation et régression, les valeurs numériques des grandeurs physico-chimiques recherchées. Les moyennes des valeurs ainsi obtenues constituent les valeurs finales caractérisant l'échantillon.

En variante, la double sélection basée sur les ellipsoïdes de confiance pour la répétabilité et la reproductibilité peut être itérée jusqu'à converger sur un ensemble de spectres « fidèles », qui seuls sont utilisés pour la régression ou la classification.

**Revendications**

1. Procédé de caractérisation d'un fluide hydraulique par application d'une technique d'analyse à un spectre d'un échantillon du fluide à caractériser pour déterminer une pluralité de grandeurs physico-chimiques dudit fluide, ledit procédé comportant l'étape a) suivante :

   a) acquisition d'un spectre d'un échantillon du fluide à caractériser, ledit fluide étant du type ester phosphate et ledit spectre comprenant un spectre d'absorption infrarouge;

   ledit procédé étant **caractérisé en ce que** l'étape a) est suivie des étapes b) et c) suivantes :

   b) calcul, par la technique d'analyse qui est une technique d'analyse statistique fonctionnelle, d'un vecteur de paramètres représentatifs dudit spectre en utilisant l'intégralité du spectre dans une ou plusieurs régions spectrales, ladite technique d'analyse statistique étant une analyse en composantes principales fonctionnelle de préférence régularisée; et
   c) détermination de la pluralité de grandeurs physico-chimiques du fluide pour caractériser ledit fluide à partir dudit vecteur de paramètres représentatifs dudit spectre.

2. Procédé selon la revendication 1, dans lequel l'application de ladite technique d'analyse statistique fonctionnelle comprend :

   - le découpage dudit spectre en une pluralité de portions ;
   - le calcul, par analyse en composantes principales fonctionnelle, d'un vecteur de paramètres représentatifs de chaque dite portion ; et
   - la concaténation des vecteurs de paramètres ainsi obtenus.

3. Procédé selon l'une des revendications 1 à 2, dans lequel, après l'application de ladite technique d'analyse statistique fonctionnelle, on détermine si ledit spectre doit être écarté par application de la méthode de Wilks audit vecteur de paramètres.

4. Procédé selon l'une des revendications 1 à 3, dans lequel ladite technique d'analyse statistique fonctionnelle est mise en oeuvre en décomposant ledit spectre sur une base de fonctions propres déterminée à partir d'une base de données de spectres de référence.

5. Procédé selon l'une des revendications 1 à 4, dans lequel les valeurs desdites grandeurs physico-chimiques sont calculées par régression multivariée à l'aide de coefficients de régression déterminés à partir d'une base de données de spectres de référence.

6. Procédé selon la revendication 5, comprenant également une opération de classification pour attribuer ledit spectre à une classe d'échantillons, lesdits coefficients de régression dépendant de ladite classe.

7. Procédé selon l'une des revendications 3 à 6, dans lequel le spectre dudit échantillon est ajouté à ladite ou au moins une dite base de données de spectres de référence.

8. Procédé selon l'une des revendications 1 à 7 comportant le fractionnement de l'échantillon à caractériser en une pluralité de sous-échantillons, l'acquisition de deux spectres d'absorption de chaque sous-échantillons de manière à obtenir une pluralité de paires de spectres, et la détermination de régions de confiance desdites grandeurs physico-chimiques par application d'un test circulaire sur les vecteurs de paramètres caractéristiques obtenus pour lesdites paires de spectres.

9. Procédé selon la revendication 8, dans lequel ledit test circulaire comprend la détermination d'un ellipsoïde de confiance de répétabilité et d'un ellipsoïde de confiance de reproductibilité, et la sélection des seuls spectres **caractérisés par** une répétabilité et une reproductibilité considérées comme suffisantes.

10. Procédé selon la revendication 9, dans lequel ladite sélection des seuls spectres **caractérisés par** une répétabilité et une reproductibilité considérées comme suffisantes est itérée.

11. Procédé selon l'une des revendications précédentes comportant également, préalablement à l'application de ladite technique d'analyse statistique fonctionnelle, une étape de prétraitement dudit spectre comprenant la soustraction d'une moyenne lissée, calculée à partir d'une base de données de spectres de référence.

12. Procédé selon l'une des revendications précédentes dans lequel, préalablement à l'application de ladite technique d'analyse statistique fonctionnelle, on détermine si ledit spectre doit être écarté par mesure de l'aire sous-tendue par un pic ou massif prédéterminé du spectre.

13. Procédé selon l'une des revendications précédentes, dans lequel lesdites grandeurs physico-chimiques comprennent au moins une grandeur parmi : la teneur en eau, un indice d'acidité, la conductivité, la viscosité, la densité, la teneur en chlore.

14. Procédé selon l'une des revendications précédentes, dans lequel ledit spectre est acquis au moyen d'un équipement embarqué sur un avion, relié à un circuit hydraulique de ce dernier.

15. Appareil de caractérisation d'un fluide hydraulique, comprenant :

   - un spectromètre (SP), pour acquérir un spectre d'un échantillon d'un fluide hydraulique à caractériser ; et
   - un moyen de traitement des données (MTD) pour déterminer une pluralité de grandeurs physico-chimiques dudit fluide par application d'une technique d'analyse audit spectre ;

   ledit appareil étant adapté pour la mise en oeuvre d'un procédé selon l'une des revendications 1 à 14.


**Patentansprüche**

1. Verfahren zur Charakterisierung einer Hydraulikflüssigkeit durch Anwendung einer Analysetechnik an einem Spektrum einer Probe der zu charakterisierenden Flüssigkeit, um eine Vielzahl von physikalisch-chemischen Größen der Flüssigkeit zu ermitteln, wobei das Verfahren den folgenden Schritt a) umfasst:

   a) Erhalten eines Spektrums einer Probe der zu charakterisierenden Flüssigkeit, wobei die Flüssigkeit vom Typ Phosphatester ist und das Spektrum ein Infrarot-Absorptionsspektrum umfasst;

   wobei das Verfahren **dadurch gekennzeichnet ist, dass** der Schritt a) von den folgenden Schritten b) und c) gefolgt wird:

   b) Berechnen, durch die Analysetechnik, die eine Technik der funktionalen statistischen Analyse ist, eines Vektors von Parametern, die für das Spektrum repräsentativ sind, indem die Gesamtheit des Spektrums in einem oder mehreren Spektralbereichen verwendet wird, wobei die Technik der statistischen Analyse eine vorzugsweise standardisierte, funktionale Hauptkomponentenanalyse ist; und
   c) Ermitteln der Vielzahl von physikalisch-chemischen Größen der Flüssigkeit, um die Flüssigkeit ausgehend von dem Vektor von Parametern zu charakterisieren, die für das Spektrum repräsentativ sind.

2. Verfahren nach Anspruch 1, wobei die Anwendung der Technik der funktionalen statistischen Analyse umfasst:

   - die Zerlegung des Spektrums in eine Vielzahl von Abschnitten;
   - die Berechnung, durch funktionale Hauptkomponentenanalyse, eines Vektors von Parametern, die für jeden Abschnitt repräsentativ sind; und
   - die Konkatenation der so erhaltenen Vektoren von Parametern.

3. Verfahren nach einem der Ansprüche 1 bis 2, wobei nach der Anwendung der Technik der funktionalen statistischen Analyse durch Anwendung der Wilks-Methode an dem Vektor von Parametern ermittelt wird, ob das Spektrum verworfen werden muss.

4. Verfahren nach einem der Ansprüche 1 bis 3, wobei die Technik der funktionalen statistischen Analyse durchgeführt wird, indem das Spektrum auf einer Grundlage von Eigenfunktionen, die ausgehend von einer Referenzspektren-Datenbank ermittelt werden, zerlegt wird.

5. Verfahren nach einem der Ansprüche 1 bis 4, wobei die Werte der physikalisch-chemischen Größen durch Mehrfachregression mithilfe von Regressionskoeffizienten, die ausgehend von einer Referenzspektren-Datenbank ermittelt werden, berechnet werden.

6. Verfahren nach Anspruch 5, das auch einen Vorgang der Klassifizierung umfasst, um das Spektrum einer Klasse von Proben zuzuordnen, wobei die Regressionskoeffizienten von der Klasse abhängen.

7. Verfahren nach einem der Ansprüche 3 bis 6, wobei das Spektrum der Probe der oder mindestens einer Referenzspektren-Datenbank hinzugefügt wird.

8. Verfahren nach einem der Ansprüche 1 bis 7, umfassend die Aufteilung der zu charakterisierenden Probe in eine Vielzahl von Teilproben, das Erhalten von zwei Absorptionsspektren von jeder Teilprobe derart, dass eine Vielzahl von Spektrenpaaren erhalten wird, und die Ermittlung von Konfidenzbereichen der physikalisch-chemischen Größen durch Anwendung eines Zirkulartests an den Vektoren von charakteristischen Parametern, die für die Spektrenpaare erhalten werden.

9. Verfahren nach Anspruch 8, wobei der Zirkulartest die Ermittlung einer Konfidenzellipse der Wiederholbarkeit und einer Konfidenzellipse der Reproduzierbarkeit und die Auswahl der einzigen Spektren, die durch eine Wiederholbarkeit und eine Reproduzierbarkeit gekennzeichnet sind, die als ausreichend erachtet werden, umfasst.

10. Verfahren nach Anspruch 9, wobei die Auswahl der einzigen Spektren, die durch eine Wiederholbarkeit und eine Reproduzierbarkeit gekennzeichnet sind, die als ausreichend erachtet werden, iteriert wird.

11. Verfahren nach einem der vorstehenden Ansprüche, das vor der Anwendung der Technik der funktionalen statistischen Analyse einen Schritt der Vorverarbeitung des Spektrums umfasst, umfassend die Subtraktion eines geglätteten Mittelwerts, der ausgehend von einer Referenzspektren-Datenbank berechnet wird.

12. Verfahren nach einem der vorstehenden Ansprüche, wobei vor der Anwendung der Technik der funktionalen statistischen Analyse durch Messung des durch eine Spitze oder ein vorgegebenes Massiv des Spektrums aufgespannten Flächeninhalts ermittelt wird, ob das Spektrum verworfen werden muss.

13. Verfahren nach einem der vorstehenden Ansprüche, wobei die physikalisch-chemischen Größen mindestens eine Größe umfassen aus: dem Wassergehalt, einer Säurezahl, der Leitfähigkeit, der Viskosität, der Dichte, dem Chlorgehalt.

14. Verfahren nach einem der vorstehenden Ansprüche, wobei das Spektrum mittels einer Ausrüstung an Bord eines Flugzeugs, die mit einem Hydraulikkreislauf dieses Letzteren verbunden ist, erhalten wird.

15. Einrichtung zur Charakterisierung einer Hydraulikflüssigkeit, umfassend:

   - ein Spektrometer (SP), um ein Spektrum einer Probe einer zu charakterisierenden Hydraulikflüssigkeit zu erhalten; und
   - ein Datenverarbeitungsmittel (MTD), um eine Vielzahl von physikalisch-chemischen Größen der Flüssigkeit durch Anwendung einer Analysetechnik an dem Spektrum zu ermitteln;

   wobei die Einrichtung für die Durchführung eines Verfahrens nach einem der Ansprüche 1 bis 14 geeignet ist.

**Claims**

1. A method for characterising a hydraulic fluid by applying an analysis technique to a spectrum of a sample of the fluid to be characterized to determine a plurality of physicochemical quantities of said fluid, said method comprising the following step a):

   a) acquiring a spectrum of a sample of the fluid to be characterized, said fluid being of the phosphate ester type and said spectrum comprising an infrared absorption spectrum;

   said method being **characterized in that** step a) is followed by the following steps b) and c):

   b) calculating, by the analysis technique which is a functional statistical analysis technique, a vector of parameters representative of said spectrum using the entire spectrum in one or more spectral regions, said functional statistical analysis technique being a preferably regularized functional principal component analysis; and
   c) determining the plurality of physicochemical quantities of the fluid to characterize said fluid from said vector of parameters representative of said spectrum.

2. The method according to claim 1, wherein applying said functional statistical analysis technique comprises:

   - partitioning said spectrum into a plurality of portions;
   - computing, by functional principal component analysis, a vector of parameters representative of each said portion; and
   - concatenating the parameter vectors thus obtained.

3. The method according to one of claims 1 to 2, wherein, after application of said functional statistical analysis technique, determining whether said spectrum should be discarded by application of Wilks' method to said vector of parameters.

4. The method according to one of claims 1 to 3, wherein said functional statistical analysis technique is implemented by decomposing said spectrum on an eigenfunction basis determined from a database of reference spectra.

5. The method according to one of claims 1 to 4, wherein the values of said physicochemical quantities are calculated by multivariate regression using regression coefficients determined from a database of reference spectra.

6. The method according to claim 5, further comprising a classification exercise to assign said spectrum to a class of samples, said regression coefficients depending on said class.

7. The method according to one of claims 3 to 6, wherein the spectrum of said sample is added to said at least one said database of reference spectra.

8. The method according to one of claims 1 to 7 comprising splitting the sample to be characterized into a plurality of subsamples, acquiring two absorption spectra from each subsample so as to obtain a plurality of pairs of spectra, and determining confidence regions of said physicochemical quantities by applying a circular test on the characteristic parameter vectors obtained for said pairs of spectra.

9. The method according to claim 8, wherein said circular test comprises determining a repeatability confidence ellipsoid and a reproducibility confidence ellipsoid, and selecting only those spectra **characterized by** repeatability and reproducibility considered sufficient.

10. The method according to claim 9, wherein said selecting only those spectra **characterized by** repeatability and reproducibility considered sufficient is iterated.

11. The method according to one of the preceding claims also comprising, prior to the application of said functional statistical analysis technique, a step of preprocessing said spectrum comprising the subtraction of a smoothed average, calculated from a database of reference spectra.

12. The method according to one of the preceding claims wherein, prior to the application of said functional statistical analysis technique, a determination is made as to whether said spectrum should be discarded by measuring the area subtended by a predetermined peak or cluster of the spectrum.

**13.** The method according to one of the preceding claims, wherein said physicochemical quantities comprise at least one of: water content, an acidity index, conductivity, viscosity, density, chlorine content.

**14.** The method according to one of the preceding claims, wherein said spectrum is acquired by means of equipment on board an aircraft, connected to a hydraulic circuit of the latter.

**15.** An apparatus for characterising a hydraulic fluid, comprising:

- a spectrometer (SP), for acquiring a spectrum of a sample of a hydraulic fluid to be characterized; and
- data processing means (DPM) for determining a plurality of physicochemical quantities of said fluid by application of an analysis technique to said spectrum ;

said apparatus being adapted for carrying out a method according to one of claims 1 to 14.

Fig. 1

Fig. 2

Fig. 3

$$y = 0{,}9999x$$
$$R^2 = 0{,}9996$$

Fig. 6

$y = 0,9997x$
$R^2 = 0,9991$

Fig. 4

$y = 0,9985x$
$R^2 = 0,9792$

Fig. 5

$$y = -1E\text{-}05x^3 + 0{,}0039x^2 + 0{,}9611x$$

$$R^2 = 0{,}6459$$

*Fig. 7*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009082418 A2

**Littérature non-brevet citée dans la description**

- **S. PAUL et al.** Chemical Contamination Sensor for Phosphate Ester Hydraulic Fluids. *International Journal of Aerospace Engineering,* vol. 2010
- **N. ROBINSON.** Monitoring oil dégradation with infrared spectroscopy. *Wearcheck Technical Bulletin,* (18
- *Lubricant Condition Monitoring Using FluidScan IR Spectroscopy Determining Lubricant Dégradation by Total Acid Number (TAN) or Oxidation by Spectro,* 03 Mai 2006, www.azom.com
- **ANA M. AGUILERA.** Using basis expansions for estimating functional PLS régression. *Chemometrics and Intelligent Laboratory Systems,* 12 Janvier 2010, vol. 104 (2), 289-305

- **ALESSANDRA BORIN.** Multivariate quality control of lubricating oils using Fourier transform infrared spectroscopy. *Journal of the Brazilian Chemical Society,* 01 Janvier 2004, vol. 15 (4), 570-576
- **LARRY WASSERMAN ; SPRINGER.** All of Nonparametric Statistics. 2006
- **P. J. GREEN ; B. W. SILVERMAN.** Nonparametric Regression and Generalized Linear Models - a roughness penalty approach. Chapman & Hall, 1994
- **J.O. RAMSAY ; B.W. SILVERMAN.** Functional Data Analysis. Springer-Verlag, 2006
- **J. P. RICHARD ; P. DEHEUVELS ; D. PIERRE-LOTI-VIAUD.** Une approche nouvelle pour l'exploitation des données chromatographiques. *Ann. Fals. Exp. Chim.,* 2000, vol. 92, 455-470